Europäisches Patentamt

European Patent Office

Office européen des brevets

(11) Publication number: **0 245 529**
**A1**

## (12) EUROPEAN PATENT APPLICATION

(21) Application number: **86106438.4**

(22) Date of filing: **12.05.86**

(51) Int. Cl.⁴: **C12N 15/00** , C12N 1/20 , C12N 9/10 , C12P 21/00

The applicant has filed a statement in accordance with Rule 28 (4) EPC (issue of samples only to experts).

The title of the invention has been amended (Guidelines for Examination in the EPO, A-III, 7.3).

(43) Date of publication of application:
**19.11.87 Bulletin 87/47**

(84) Designated Contracting States:
**AT BE CH DE FR GB IT LI LU NL SE**

(71) Applicant: **Max-Planck-Gesellschaft zur Förderung der Wissenschaften e.V.**
**Bunsenstrasse 10**
**D-3400 Göttingen(DE)**

(72) Inventor: **Keck, Wolfgang, Dr**
**Schlossgartenstrasse 27**
**D-7400 Tübingen 7(DE)**
Inventor: **Betzner, Andreas, Dipl. Biol.**
**Rossbergstrasse 48**
**D-7400 Tübingen(DE)**
Inventor: **Schwarz, Uli, Prof. Dr**
**Vöchtingstrasse 1**
**D-7400 Tübingen(DE)**

(74) Representative: **Vossius & Partner**
**Siebertstrasse 4 P.O. Box 86 07 67**
**D-8000 München 86(DE)**

(54) **Recombinant DNA vector encoding a lytic bacterial transglycosylase, and its use in the preparation of 1,6-anhydromuramyl peptides.**

(57) The subject matter of the invention is the production of lytic bacterial transglycosylases by recombinant DNA technology. The genes encoding the membrane bound and the soluble lytic transglycosylase of Escherichia coli were cloned into a high copy number plasmid suitable for overproduction of the enzymes. The transglycosylases obtained are useful for the digestion of murein to produce 1,6-anhydromuramyl peptides.

EP 0 245 529 A1

**"Recombinant DNA vector carrying the structural gene for a lytic bacterial transglycosylase, method for large scale production of the transglycosylase and its use for the preparation of 1,6-anhydromuramyl peptides".**

This invention relates to recombinant DNA vector molecules which carry a DNA sequence encoding a lytic bacterial transglycosylase, for example a lytic transglycosylase from Escherichia coli. More specifically, the recombinant DNA molecules described here contain the structural gene for the membrane bound lytic transglycosylase (mlt) or the soluble lytic transglycosylase (slt) from Escherichia coli. The structural gene encoding a lytic bacterial transglycosylase is cloned into a high copy number plasmid for propagation in a suitable host-strain for overproduction of the gene-product, due to an increased number of gene copies. The enzymes can be purified and used for complete digestion of murein to produce 1,6-anhydromuramyl peptides. These anhydromuramyl peptides are known to promote slow-wave sleep in different animals. The composition and structure of these anhydro-compounds are identical with the human urinary sleep-promoting factor.

The invention is more specifically dealing with (1) the construction of a representative E. coli gene library into a suitable prokaryotic vector, (2) selection of clones overproducing either the slt-or mlt-gene product, (3) caracterization of the recombinant DNA by restriction analysis, (4) identification and localisation of the two structural genes (slt, mlt) within the recombinant DNA, (5) subcloning of the two genes and overproduction of the two transglycosylases, (6) immunological, biochemical and physical characterization of the purified transglycosylases.

Murein (synonyma: peptidoglycan, mucopeptide) and muropeptides cause a variety of biological activities in mammalian organisms and there is evidence that they are intrinsic modulators of physiological processes within the mammalian host (S. Zhai and M.L. Karnovsky 1984, Infect. Immun. 43: 937-941). Several natural and synthetic muropeptides are, depending on their structure, active as slow-wave sleep inducers, as pyrogens, immunoadjuvants, immunomodulators and as promotors of nonspecific host resistance against viral and bacterial infections (J.M. Krueger et al. 1984, J.Biol.Chem. 259 : 12659-12662).

Recently, sleep promoting factors were isolated from brain of sleep deprived rats and human urine which induced slow-wave sleep in rabbits, rats , mice and cats (J.M. Krueger et al. 1982, J.Biol.Chem. 257: 1664-1669; H.Nagasaki et al. 1980, Brain Res. 192: 276-280). The characterization of the compounds revealed that the structure of the factors is identical with that of monomers from bacterial murein (S.A. Martin et al. 1984, J.Biol.Chem. 259: 12652-12658). Synthetic and isolated muropeptides from bacterial sources were also somnogenic (J.M. Krueger et al. 1984, J.Exp.Med. 159: 68-76).

Studies on the structural-functional relationship of the muropeptides showed that stereospecificity and the presence of the muramic acid residue were required for the expression of the somnogenic activity.

The muramic acid in the most active compounds has no reducing end but a 1,6-anhydrobond (S.A. Martin et al. 1984, J.Biol. Chem. 259: 12652-12658). It was further observed that the compounds were inactivated by amidation of carboxyl groups. Hydrolysis of the amide groups restored the somnogenic activity. This amidation-deamidation reaction might be the physiological switch for the somnogenic compounds within the brain (J.M. Krueger et al. 1984, J.Exp.Med. 159: 68-76). It was suggested that interleukin-1 might be involved in the mechanism of action of the somnogenic muropeptides. Interleukin-1 is produced by astrocytes of the central nervous system and induces similiar but faster sleep effects as the muropeptides (J.M. Krueger et al. 1984, Am.J.Physiol. 246: R 994 - R 999).

Involvement of murein and muropeptides in the induction of arthritis is another interesting aspect of the murein metabolism in mammalian organisms. It is possible to induce experimental arthritis in Lewis rats by intradermal injection of murein or muropeptides, including 1,6-anhydro compounds (I. Ginsburg et al. 1984, Isr.J.Med.Sci. 20: 470). In gonococcal disease this might be accomplished by a release of murein and muropeptides through the action of bacterial or host-derived hydrolases.

The murein and muropeptides involved in the described physiological processes of mammalian organisms are of bacterial origin. A rather extensive murein metabolism must take place in the host organisms, involving enzymes of bacterial and/or mammalian origin.

It appears that the 1,6-anhydro compounds show the strongest physiological effects: they are the most potent somnogens and have significant arthropathic activity. Cerebral intraventricular infusion of 1 picomole of the 1,6-anhydromuramyl tetrapeptide into rabbits was sufficient to induce prolonged excess slow-wave sleep (J.M. Krueger et al. 1984, J. Biol. Chem. 259: 12659-12662). These 1,6-anhydromuramyl peptides are degradation products of murein. Bacterial transglycosylases degrade murein completely into monomeric and dimeric 1,6-anhydromuramyl peptides (J.V. Höltje et al. 1975, J. Bacteriol. 124 : 1067-1076).

In Escherichia coli two different lytic transglycosylases are described, purification procedures are established and the purified enzymes are well characterized (W. Keck et al. 1985, Eur.J.Biochem. 148: 493-497). Both transglycosylases can be used for enzymatic production of the biological active 1,6-anhydromuramyl peptides from intact murein sacculi. The alternative production of these 1,6-anhydro compounds by chemical synthesis must deal with all the problems of sugar chemistry, like the very low yield in a multistep synthesis etc.

The separation and isolation of the 1,6-anhydro muropeptides by HPLC is established (B. Glauner and U. Schwarz 1983, In "The Target of Penicillin", pp 29-34, de Gruyter, Berlin, New-York). A serious problem for large scale production of 1,6-anhydro compounds is the rather low amount of enzyme present in the E. coli wildtype cells.

It is the object of the invention to provide a process for preparing and obtaining lytic bacterial transglycosylases in an amount which is high enough for their use in a large scale production of 1,6-anhydromuramyl peptides.

This object is achieved in accordance with the invention by the cultivation of a microorganism which is transfected with a recombinant DNA vector comprising a DNA sequence encoding a lytic bacterial transglycosylase.

The subject matter of the invention, therefore, is a recombinant DNA vector comprising a DNA sequence encoding a lytic bacterial transglycosylase.

Another subject matter of the invention is a host cell (microorganism) which is transformed with the said recombinant DNA vector.

A further subject matter of the invention is a process for large scale production of a lytic bacterial transglycosylase which is characterized in that

a) a DNA sequence encoding the lytic bacterial transglycosylase is introduced into a high copy number plasmid,

b) a host cell is transformed with the resulting recombinant DNA vector,

c) the transformed host cell is cultivated for overproduction of the lytic bacterial transglycosylase, and

d) the lytic bacterial transglycosylase is recovered from the cell extracts.

Finally, the invention also comprises the use of the lytic transglycosylase obtained according to the above process for the production of 1,6-anhydromuramyl peptides.

According to a specific embodiment of the invention transglycosylase overproducing E. coli strains were constructed, from which a sufficient amount of the enzymes can be isolated. The overproduction of the transglycosylases was accomplished by isolation and cloning of the structural gene for the soluble and the membrane bound lytic transglycosylase (slt, mlt) onto a temperature inducible runaway replication plasmid. For the isolation of the genes an Escherichia coli gene library was established using an expression vector with temperature inducible copy number (J.E.L. Larsen et al. 1984, Gene 28 : 45-54). The clones were screened after temperature induction with specific antibodies against the two transglycosylases by the peroxidase anti-peroxidase immunochemical staining technique for the overproduction of the soluble or the membrane bound lytic transglycosylase , respectively. Two individual clones, one for each transglycosylase were isolated and their recombinant plasmids were shown to be different by restriction analysis. Both genes (slt, mlt) were localized precisely on the cloned chromosomal fragments and their direction of transcription was determined by subcloning and deletion mapping. The specific detection of truncated proteins by immunoblotting techniques, expressed from deletion plasmids, verified that we are really dealing with the structural genes for the two lytic transglycosylases.

The invention described in this application will be explained in detail with reference to the isolation and cloning of the two structural genes for the two lytic murein-transglycosy lases from E. coli and the overproduction of the two enzymes. It allows to isolate sufficient amounts of the enzymes for the large scale production of 1,6-anhydromuramyl peptides by enzymatic degradation of bacterial murein.

These 1,6-anhydromuramyl peptides accomplish a rather broad spectrum of very interesting physiological effects in mammalian organisms, like sleep induction, arthropathic activity and stimulation of the immune system.

Compared to the production of 1,6-anhydro compounds by conventional chemical synthesis or by isolation from brain of sleep deprived animals or human urine this invention allows an unrivalled simple production of the biological active 1,6-anhydromuramyl peptides.

## Construction of the gene library in pWK12.

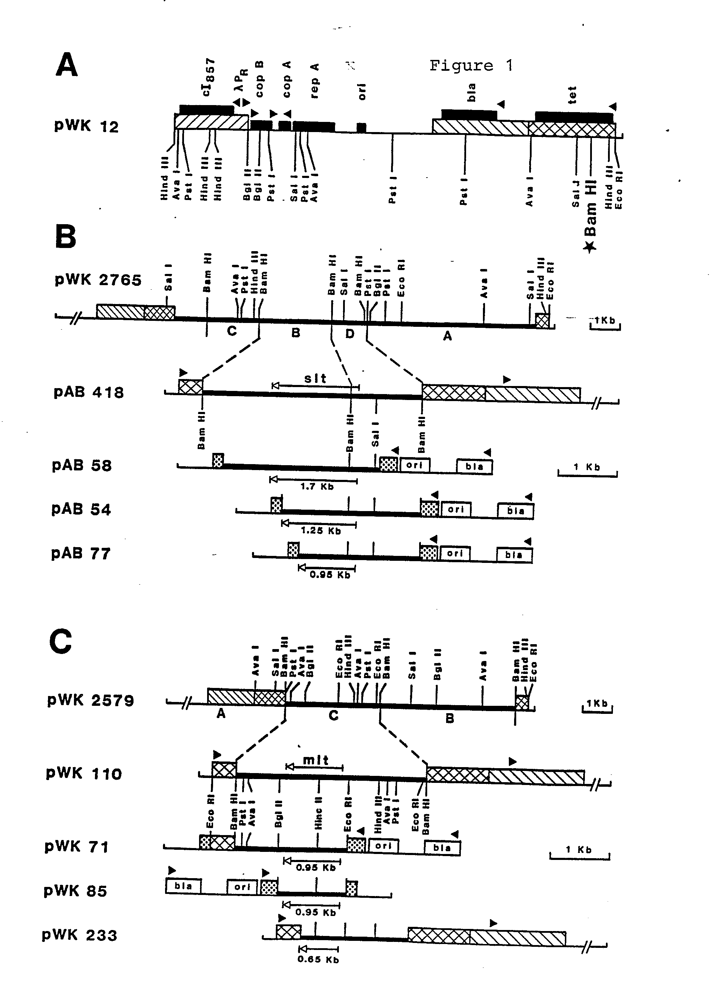

The molecular cloning of the structural genes for murein hydrolases in E. coli might be a difficult task due to the increased gene dosage and the overproduction of the plasmid-coded gene products. It was not clear whether the cells would be able to tolerate an increased gene copy number for one of the two lytic transglycosylases. To diminish this problem, the low copy number plasmid pOU71 with temperature-inducible copy number was chosen as a basis for the construction of pWK12, which carries in addition to the β-lactamase gene a tetracycline resistance gene with a Bam HI-cloning site (Fig. 1A). The temperature-inducible amplification of the plasmid-copy number is paralleled by an increased production of plasmid-coded proteins (I.E.L. Larsen et al. 1984, Gene 28: 45-54). These properties make the plasmid pOU71 and its derivative pWK12 a suitable vector for establishing a gene library. The identification of a specific clone from such a gene library is facilitated by the fast overproduction of plasmid coded gene products in the first 3 hours after temperature induction. It was expected that within a gene library, constructed with this vector, the clones for potentially detrimental genes like the two lytic transglycosylases could be kept stable at 30°C and could be identified after temperature induction by screening with specific antibodies for overproduction of the transglycosylases.

Chromosomal DNA was prepared from strain W3110 and partially digested with Sau3AI to generate overlapping fragments of various sizes, which were separated on an agarose-gel to isolate 8-12 Kb fragments. Insertion of the fragments into the single BamHI site of pWK12 was mediated by T4 DNA-ligase. After ligation, the DNA mixture was used for transformation of HB101 cells. Ampicillin resistant but tetracycline sensitive transformants were picked for the gene library. From 100 randomly chosen clones plasmid DNA was isolated and the size of the inserted fragments was determined after cleavage with restriction endonuclease Aval. The average size of the inserted chromosomal fragments is 11,3 Kb. This gene library was stored as glycerol cultures at -70°C.

## Immunological screening for transglycosylase overproducing clones.

The gene library was screened with the peroxidase antiperoxidase immunochemical staining technique for the identification of clones which overproduce one of the two transglycosylases, respectively. This screening program was based on the possibility to identify colonies, which overproduced one of the two transglycosylases due to the temperature-induced amplification of plasmid coded transglycosylases. By the immunochemical staining of the nitrocellulose filters after incubation with the specific antisera against both transglycosylases, respectively, one positive clone for each transglycosylase species was identified.

## Characterization of recombinant clones.

The two positive clones were numbered according to their position on the microtiter plates. The clone HB101 (pWK2765) gave a positive response with antiserum against the soluble lytic transglycosylase (Slt), whereas the clone HB101 (pWK2579) was positive for the membrane-bound lytic transglycosylase (Mlt). The clones HB 101 (pWK 2765) and HB-101 (pWK 2579) were deposited with Deutsche Sammlung für Mikroorganismen (DSM) under the accession numbers DSM 3711 and DSM 3712, respectively.

Plasmid DNA from both clones was purified and analysed by restriction-endonuclease mapping. The recombinant plasmids pWK2765 and pWK2579 contain chromosomal fragments of 12.1 Kb and 7.6 Kb, respectively. The restriction pattern of the two cloned chromosomal fragments showed no similarity with each other, which means that the two fragments were derived from two different regions of the E. coli chromosome (Fig. 1B,C).

## Subcloning of the slt-and mlt-gene.

The localisation of the two genes (slt, mlt) on the two cloned chromosomal fragments was further investigated by subcloning various restriction endonuclease fragments into the vectors pWK12 or pUC9 (Fig. 1). The resulting transformants were tested again for the overproduction of the transglycosylases by the peroxidase anti-peroxidase (PAP) immunochemical staining technique. The first crude mapping of the slt gene was done by isolation of the BamHI fragments from the original plasmid pWK2765 and by cloning of these fragments singly and in combinations of two into the vector pWK12. Subclones were only positive

4

with the PAP immunochemical staining technique when the BamHI fragments B and D were subcloned in the original orientation (Fig. 1: pAB418). The fragment B alone did not lead to overproduction of the slt gene product. For a more precise localisation of the coding region a mixture of Sau 3AI fragments (0.9-3 Kb) was isolated after limited digestion of the slt containing Hind III-Pst I-fragment of pWK2765. Sau 3AI fragments were ligated into the BamHI site of pUC9 and the ligation mixture was used to transform JM83 cells. β-Galactosidase negative colonies from ampicillin-containing agar plates were screened with the peroxidase-anti-peroxidase immunochemical staining technique for the overproduction of the slt gene product. Plasmid DNA from 7 overproducing clones was isolated and analysed by restriction mapping, three of the plasmids are presented in figure 1B (pAB58, pAB54, pAB77).

The mlt-gene was localized on pWK2579 by subcloning various fragments (Fig. 1C). The first crude mapping was done by isolation and subcloning of the BamHI fragments B or C from pWK2765 into the BamHI site of pWK12.

After screening the transformants for overproduction of the mlt-gene product with Mlt-specific antibodies in the PAP-assay, we analysed the plasmid DNA from positive clones and found the fragment C in pWK110 leading to overproduction of the membrane-bound lytic transglycosylase. Further subcloning of smaller fragments into pUC9, like the BamHI-EcoRI 1.9 Kb fragment (pWK71) or the BglII-EcoRI 1.15 Kb fragment (pWK85), revealed that these fragments contain all the information for the overproduction of the mlt-gene product (Fig. 1C).

Finally, an incomplete digestion of pWK110 with Sau3AI and subcloning of the fragments into the BamHI-site of pWK12 led to the plasmid pWK233, from which the membrane-bound transglycosylase was overproduced in truncated form (PAP assay).

## Expression of the cloned slt-and mlt-gene.

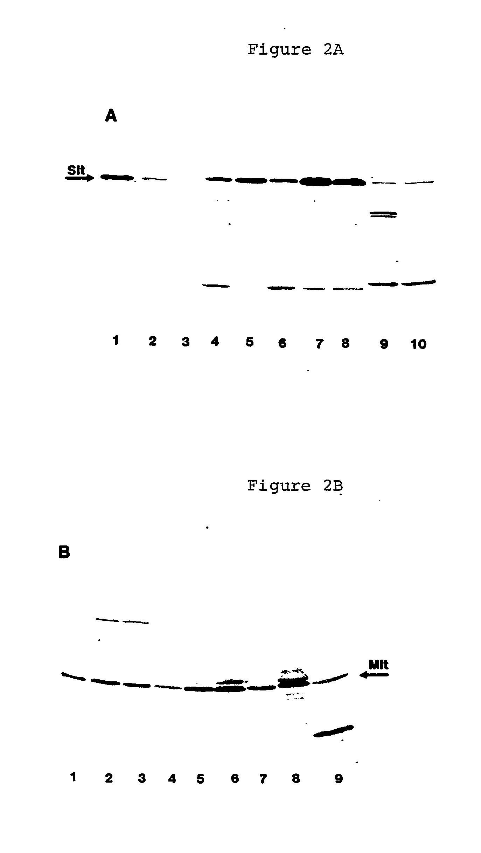

All the subclones, represented by the plasmids in Figures 1B,C were tested for the overproduction of the soluble or the membrane-bound lytic transglycosylase, respectively. The cells were grown with aeration in 20 ml LB-broth in the presence of ampicillin at 30°C. Clones carrying plasmids with temperature inducible runaway replication were shifted at an $OD_{578}$ of 0.3 to 42°C for 90 min. The other clones were kept at 30°C to an $OD_{578}$ of 0.6. The cultures were cooled on ice, cells were harvested by centrifugation and crude extracts (the cytoplasm or membranes) were obtained from the clones and the host strains as described earlier (W. Keck et al. 1985, Eur.J.Biochem. 148: 493 - 497). From each crude extract 150 ug of protein was loaded onto a 10% sodium dodecyl sulphate polyacrylamide gel. Proteins were electrophoretically separated and transferred onto nitro-cellulose filters for the specific staining of the soluble or the membrane-bound lytic transglycosylase by the immunochemical PAP staining technique (Figs. 2A,B).

The original gene library clone HB 101 (pWK2765) as well as the subclone with the plasmid pAB418 led under these conditions to a 10 fold overproduction of the soluble transglycosylase compared to the wildtype expression level of the host strain HB101 (Fig. 2A). The overproduction of the enzyme was quantitated by a dilution series of the crude extract and scanning of the immunoblotts.

The precise localisation of the slt-gene and its direction of transcription were obtained by constructing the plasmids pAB58, pAB54 and pAB77 as described before. The subclone JM83 (pAB58) expressed the enzyme as an intact protein (relative molecular mass 65 000) at a 10 fold level compared to the wildtype level of JM83. The two other subclones JM83 (pAB54) and JM83 (pAB77) expressed the normal level of the intact protein from their chromosomal gene-copy and in addition at higher rate a truncated polypeptide with a relative molecular mass of about 48 000 or 36 000, respectively (Fig. 2A, lane 8-10). Since in these two recombinant plasmids the cloned chromosomal fragments are shortened from the same end, both position and orientation of the transcriptional unit for the slt-gene (Fig. 1B) were obvious. The expression of truncated polypeptide chains from the plasmids pAB54 and pAB77 proved that we are really dealing with the structural gene for the soluble lytic transglycosylase (slt). The protein with a relative molecular mass of 29000 was unspecifically stained even after incubation with control-serum (data not shown). The overproduction of the membrane-bound lytic transglycosylase (Mlt) was assayed as described for Slt (see above) but using membrane extracts and a Mlt-specific antiserum. The original gene library clone HB101 (pWK 2579) and the subclone HB101 (pWK110), which contained the Bam HI-fragment C (3.2 Kb) showed after temperature induction a 10 fold increased level of the Mlt compared to the wildtype level in the host strain HB101. Further subcloning of smaller fragments into the vector pUC9 led to the plasmid pWK71 and pWK85.

5

Transformants carrying one of these two plasmids were rather instable and grew very slowly which might be due to the increased mlt-gene copy number of about 20-30 copies per cell. For the membrane-bound lytic transgycosylase with a relative molecular mass of 35 000, a coding region of about 0.95 Kb is needed. The cloned chromosomal fragment in pWK85 (1.15 Kb) still carried the complete mlt-gene and led to a rather high overproduction of the Mlt (Fig. 2B, lane 8). The direction of transcription for the mlt-gene was determined with the plasmid pWK233 from which a truncated protein is translated with a relative molecular mass of 25 000 (Fig. 2B, lane 9). This plasmid compared with pWK85 is shortened at only one side of the chromosomal fragment, which means that the lost DNA fragment is coding for about 66 amino terminal amino acids of Mlt, it also defines the direction of transcription for the mlt-gene as shown in Fig. 1C.

The staining of the protein with a relative molecular mass of 72 000 could even be detected after incubation with control-serum (data not shown).

Transglycosylase activity in crude extracts from Slt and Mlt overproducing clones

The enzymatic activity of both transglycosylases is dramatically inhibited in cell crude extracts. The measurable activity does not reflect the actual amount of the two enzymes present (W. Kusser and U. Schwarz 1980, Eur.J.Biochem. 103: 277 - 281). It is completely unknown whether this inhibition has any physiological relevance in controlling the transglycosylases to prevent cell lysis (J.V. Höltje and U. Schwarz 1983, in "The Target of Penicillin"; pp 185-190, de Gruyter 6 Co., Berlin, New York).

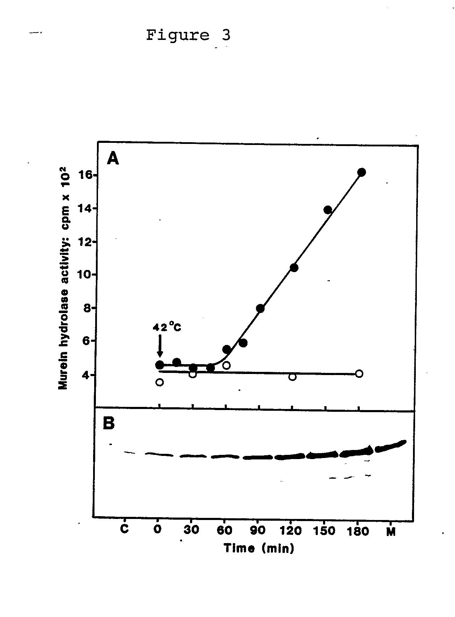

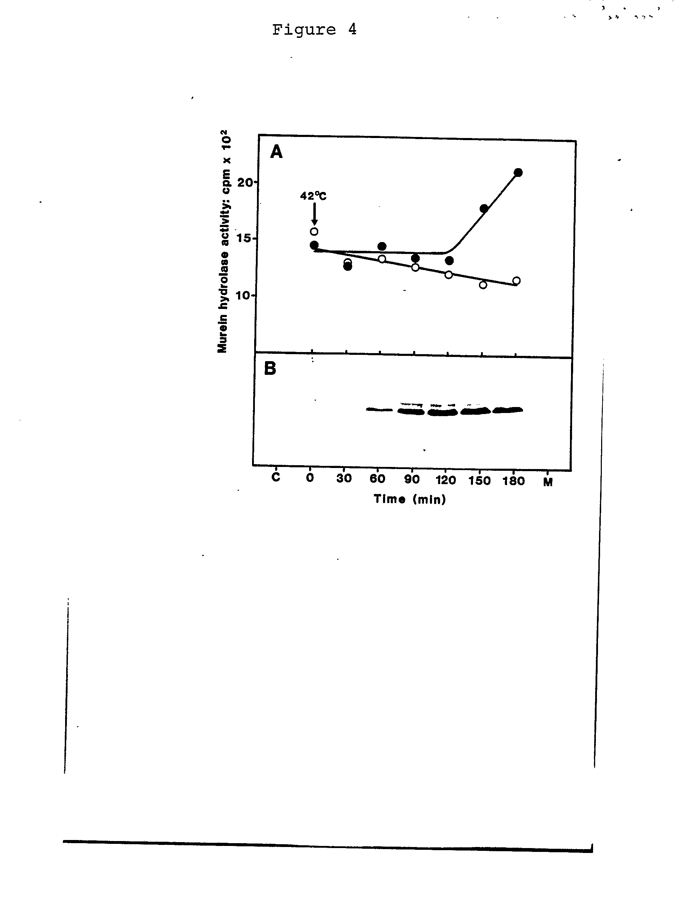

In both transglycosylase-overproducing clones the increase of the transglycosylase protein content and the enzymatic activities were assayed at various times of induction (Figs. 3A, 4A).

Already 30 min after the HB101 (pWK2765) or HB101 (pWK2579) cells were shifted to 42°C the amount of Slt-protein or Mlt-protein, respectively were slightly increased and the cells continued to overproduce the proteins during 3 hours after the temperature shift (Figs. 3B,4B). The measurable transglycosylase activity in the soluble fractions from HB101 (pWK2765) increased with a delay of 60 min after temperature induction and reached a 4-fold level after 3 hours (Fig. 3A), whereas the transglycosylase activity in the membrane extracts from HB101 (pWK2579) stayed constant for the first 2 hours after temperature induction and increased during the third hour only by a factor of 1.5 (Fig. 4A). Longer incubation times at 42°C were lethal to the plasmid-carrying cells, due to the high amplification rate of the plasmid DNA (J.E.L. Larsen et al. 1984, Gene 28:45-54).

Description of the drawing

Fig. 1:

Restriction maps of the cloning vector pWK12 (A), the two plasmids from the gene library, pWK2765 (B) carrying the slt-gene or pWK2579 (C) the mlt-gene, and the localisation of the two genes by deletion-mapping. The construction of the vector pWK12 on the basis of the plasmid pOU71 is described in Materials and Methods. The single BamHI site (*) in pWK12 was used for cloning chromosomal Sau3AI-fragments to establish the E. coli gene library (Material and Methods). The two structural genes (slt;mlt) for the soluble or the membrane-bound lytic transglycosylase were localised and the direction of transcription was determined by subcloning deleted chromosomal fragments into the BamHI-site of pWK12 or the multiple-cloning sites of pUC9, as described in the Results section.

▨ DNA-derived from λ-phage; ◩ Tn3; ▩ pBR322; ▬ gene; ▦ lacZ´ ; ▬ E. coli chromosomal DNA; ▶ promoter

Fig. 2:

Expression of the cloned slt-and mlt-gene in various clones. The different clones were grown and crude extracts of the soluble-(A) and the membrane-fraction (B) were prepared as described above. The enzymes Slt and Mlt were detected with the immunochemical PAP-staining technique after electrophoretic transfer of the proteins from a 10% sodium dodecylsulphate polyacrylamide-gel onto nitrocellulose. A: Purified Slt (0,25ug) was loaded as a marker (1). Crude cytoplasmic extracts (each 150 $\mu$g) was loaded onto the polyacrylamide-gel from the host strain HB 101 (2), JM 83 (3), the gene library clone HB 101 pWK 2765 grown at 30° C (4) and shifted to 42 °C for 90 min (5), the subclone HB 101 pAB 418 grown at 30 °C (6) and shifted to 42 °C for 90 min (7), the host strain JM 83 carrying the plasmid pAB 58 (8), pAB 54 (9) and pAB 77 (10). The later two clones express truncated polypeptide chains. The incubation of the nitrocellulose filter with the Slt-specific antiserum was carried out for 10 min with a serum dilution of 1:500. B: Purified Mlt (0,2 $\mu$g) was loaded as a marker (1). Crude membrane extracts (each 100 $\mu$g) were loaded from the two host strains HB 101 (2) and JM 83 (3), the gene library clone HB 101 pWK 2579 grown at 30 °C (4) and shifted to 42 °C for 90 min (5), the subclones HB 101 pWK 110 (6), JM 83 pWK 71 (7), JM 83 pWK 85 (8) and HB 101 pWK 233 (9). The last clone expresses a truncated polypeptide chain. The nitrocellulose filter was incubated with the Mlt-specific antiserum at a dilution of 1:400 for 20 min and further processed. The additional stained bands were detected even after incubation with control sera.

Fig. 3:

Overproduction of the soluble lytic transglycosylase (Slt) in HB101 (pWK2765) cells after temperature induced plasmid amplification. The bacteria were grown and samples prepared as described above. The enzymatic activity of the Slt was measured as murein hydrolase activity in crude extracts from HB101 (-o-); compared with the same strain carrying the plasmid pWK2765 (-●-). The amount of expressed Slt-protein is shown as immunochemical PAP-staining of the protein after electrophoretic transfer from polyacrylamide gel (10 %) onto nitrocellulose. As a control the crude extract from the host strain HB101 was used (C). Purified Slt (0.25 $\mu$g) was loaded onto the gel as marker (M).

Fig. 4:

Overproduction of the membrane-bound lytic transglycosylase (Mlt) in HB101 (pWK2579) cells after temperature induced plasmid amplification. The bacteria were grown and samples prepared as described above. The enzymatic activity of the Mlt was measured as murein hydrolase activity in crude membrane extracts from HB101 (-o-); compared with the same strain carrying the plasmid pWK2579 (-●-). The amount of expressed Mlt-antigen is shown as immunochemical PAP-staining of the protein after electrophoretic transfer from polyacrylamide gel (10%) onto nitrocellulose. As a control, membrane extract from the host strain HB 101 was used (C). Purified Mlt (0,2 $\mu$g) was loaded onto the gel as marker (M).

MATERIALS AND METHODS

Bacterial strains, plasmids and media.

For the isolation of chromosmal DNA the E. coli K12 wildtype strain W3110 was used (W. Weidel and H. Pelzer 1964, Adv.Enzymol. 26: 193-232). HB101 (F⁻, hsdS20 ($r_B$, $m_B$), recA13, ara-14, proA2, lacY1, galK2, rpsL20 (Sm$^r$), xyl-5, mtl-1, supE44, λ⁻) was the recipient for all transformations with pWK12 and pBR322 and their derivatives (H.W. Boyer and D. Roulland-Dussoix 1969, J.Mol.Biol. 41: 459-472). JM83 (Δ(lac, pro), thi, strA, Ø80 d lac Z ΔM15) was used as a recipient for pUC9 and its derivatives (J. Vieira and J. Messing 1982, Gene 19: 259-268). Bacteria were grown in L-broth. For the selection of transformants L-broth and agar plates were supplemented with either 50 $\mu$g/ml ampicillin or 25 $\mu$g/ml tetracycline. The selection of transformants for pWK12 and its derivatives at 30°C was carried out with media containing half of the above mentioned concentration of antibiotics.

## Restriction endonucleases and other enzymes.

The restriction endonucleases AvaI, BamHI, BglII, EcoRI, HincII, HindIII, KpnI, PstI, SalI, Sau3AI, SmaI and XhoI; T4 DNA ligase, DNA-polymerase I (Klenow fragment), alkaline phosphatase from calf intestine and DNA exonuclease Bal31 were obtained from Boehringer Mannheim. DNase I from pancreas and lysozyme were purchased from Serva. The soluble and membrane-bound murein transglycosylases were purified as described earlier (W. Keck and U. Schwarz 1979, J.Bacteriol. 139: 770-774; W. Keck et al. 1985, Eur.J.Biochem. 148: 493-497).

## Antisera and antibodies.

The specific antisera against the soluble and the membrane-bound lytic transglycosylase, respectively were kindly provided by F.B. Wientjes (W. Keck et al. 1985, Eur.J.Biochem. 148: 493-497). The goat anti-rabbit antiserum was a gift from H. Schwarz. Peroxidase anti-peroxidase (rabbit) was from Miles and the goat F (ab') 2-anti rabbit IgG peroxidase conjugate was obtained from Medac.

## Chemicals

Agarose for electrophoresis was obtained from Pharmacia. Ampicillin was a gift from Bayer. Amidoblack 10-B, coomassie brilliant blue R250, isopropyl-$\beta$-D-thiogalactopyranosid (IPTG), 5-brom-4-chlor-3-indoxyl-$\beta$-D-galactosid (X-Gal), tetracycline, Triton X-100 and acrylamide were from Serva. Sodium dodecyl sulphate was obtained from BDH. Nitrocellulose filters were from Schleicher & Schüll. Tween 20 and 4-chloro-1-naphtol were purchased from Sigma. All other chemicals used were obtained from Merck.

## Isolation of chromosomal DNA, plasmid DNA and DNA fragments, treatment with restriction endonucleases, ligation and transformation.

Chromosomal DNA was isolated following the procedure of H. Saito and K.-J. Miura (1963, Biochim.Biophys.Acta 72: 619-629). For large scale preparation of plasmid DNA the plasmids were amplified either by chloramphenicol treatment or by temperature shift of the culture for 2 hrs to 42°C and isolated as described by G.N. Godson and D. Vapnek (1973, Biochim. Biophys.Acta 299: 516-520). Small scale plasmid DNA preparations were done essentially according to H.C. Birnboim and J. Doly (1979, Nucleic Acids Res. 7: 1513-1523). The caracterization of plasmid DNA by restriction analysis was carried out with 0.5 - 1.2 % agarose gels in 90 mM Tris-borate buffer, pH 8.3; 2.5 mM EDTA, using lambda DNA restricted with Eco RI and Hind III as molecular weight standard. DNA-fragments generated by restriction endonuclease cleavage were isolated from agarose gels as described by G. Dretzen et al. (1981, Analyt.Biochem. 112: 295-298). Digestion of DNA with restriction endonucleases, Bal31 exonuclease, alkaline phosphatase and ligation of DNA fragments with T4 polynucleotide ligase were done following the recommendations of the enzyme supplier. Transformation of cells with plasmid DNA was done according to M. Mandel and A. Higa (1970, J.Mol.Biol. 53: 154-162).

## Example

### A) Construction of the cloning vector pWK12.

The low copy number plasmid pOU71 with temperature inducible runaway replication was the basis for establishing the E. coli gene library. This plasmid was constructed and described in detail by J.E.L. Larsen et al. (1984, Gene 28: 45-54). For easier selection of recombinant clones the 0.5 Kb Bam HI-EcoRI fragment was exchanged against the 1.45 Kb BalI-EcoRI fragment from pBR322, carrying the tetracycline resistance gene (tet) to get the plasmid pWK12 (Fig. 1A). The plasmid pOU71 was restricted with Bam HI, the 5'-protruding ends were made blunt by S1-Nuclease digestion and after restriction with Eco RI a 6 Kb fragment was isolated from an agarose gel. The 1.45 Kb fragment from plasmid pBR322, carrying the tet-gene was isolated after Eco RI and Bal 1 digestion. The two isolated fragments were ligated by T4 polynucleotide ligase and HB 101 transformants were selected on ampicillin and tetracycline containing agar-plates at 30°C.

## B) E. coli gene library

High molecular weight chromosomal DNA, isolated from the strain W3110, was partially digested with Sau3AI and 8-12 Kb fragments were isolated. The pooled fragments were cloned into the BamHI-site within the tetracycline resistance gene of pWK12 (Fig. 1). After selecting on agar plates for ampicillin resistant and tetracycline sensitive clones at 30°C, 4500 wells of microtiter plates were inoculated with single colonies and incubated in the presence of ampicillin (25 µg/ml) at 30°C overnight. Glycerol as added to a final con centration of 10 % and the microtiter plates were stored at -70°C.

## C) Immunological screening.

The clones of the gene library were transferred from the microtiter plates with the 96 needles of a metal replicating block onto ampicillin containing agar-plates. The plates were incubated overnight at 30°C and then for 2-3 hours at 42°C to amplify plasmid DNA and plasmid coded gene products. The colonies were replica transferred onto nitrocellulose filters. For lysis of the baceria and the following incubation steps with the specific antisera we followed essentially the protocol of D.M. Helfman et al. (1983, Proc.Natl.Acad. Sci. USA 80: 31-35). Overproducing clones for the two lytic transglycosylases were individually identified by the peroxidase anti-peroxidase (PAP) immunochemical staining technique (L.A. Sternberger et al. 1970, J.Histochem.Cytochem. 18: 315-333).

## D) Electrophoresis of proteins, blotting and immunochemical staining of proteins.

Proteins were separated by sodium dodecyl sulphate polyacrylamide gel-electrophoresis according to B. Lugtenberg et al. (1975 FEBS-Lett. 58: 254-258). Gels were stained with Coomassie Brilliant Blue R-250. The electrophoretic transfer of proteins from polyacrylamide gels onto nitrocellulose was done as described by W.N. Burnette (1981, Analyt.Biochem. 112: 195-203).

The nitrocellulose filters were saturated with Tween 20 and the proteins were stained specifically with the peroxidase anti-peroxidase (PAP) immunochemical staining technique using antisera specific for the soluble or membrane-bound transglycosylase, respectively (B. Batteiger et al. 1982, J.Immun. Meth. 55: 297-307).

## E) Analytical methods.

The enzymatic activities of both lytic transglycosylases were essentially assayed as described earlier (J.V. Höltje et al. 1975, J.Bacteriol. 124: 1067-1076), and the reaction products were analysed by high-performance liquid chromatography (B. Glauner and U. Schwarz 1983, in "The Target of Penicillin" pp 29-34, de Gruyter & Co., Berlin, New York) Protein concentrations were determined following the method of O.H. Lowry et al. (1951, J.Biol.Chem. 193-275).

## Claims

1. A recombinant DNA vector comprising a DNA sequence encoding a lytic bacterial transglycosylase.

2. The vector of claim 1 wherein the DNA sequence encodes the membrane bound lytic transglycosylase of Escherichia coli.

3. The vector of claim 1 wherein the DNA sequence encodes the soluble lytic transglycosylase of Escherichia coli.

4. The vector of any of claims 1 to 3 which is derived from a high copy number plasmid.

5. The vector of claim 2, plasmid pWK2579.

6. The vector of claim 3, plasmid pWK2765.

7. A host cell transformed with the recombinant DNA vector of any of claims 1 to 6.

8. A process for large scale production of a lytic bacterial transglycosylase characterized in that

a) a DNA sequence encoding the lytic bacterial transglycosylase is introduced into a high copy number plasmid,

b) a host cell is transformed with the resulting recombinant DNA vector,

c) the transformed host cell is cultivated for overproduction of the lytic bacterial transglycosylase, and

d) the lytic bacterial transglycosylase is recovered from the cell extracts.

9. The process of claim 8 wherein the lytic bacterial transglycosylase is a lytic transglycosylase from Escherichia coli.

10. The use of the lytic transglycosylase obtained according to claims 8 or 9 for the production of 1,6-anhydromuramyl peptides.

Figure 1

Figure 2A

Figure 2B

Figure 3

Figure 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | EUROPEAN JOURNAL OF BIOCHEMISTRY, vol. 148, 1985, pages 493-497, FEBS; W. KECK et al.: "Comparison of two hydrolytic murein transglycosylases of Escherichia-coli" <br> * Whole document * | 1-9 | C 12 N 15/00 <br> C 12 N 1/20 <br> C 12 N 9/10 <br> C 12 P 21/00 |
| Y | PROCEEDINGS OF THE NATIONAL ACADEMY OF SCIENCES USA, vol. 80, March 1983, pages 1194-1198; R.A. YOUNG et al.: "Efficient isolation of genes by using antibody probes" <br> * Whole document * | 1-9 | |
| A,D | JOURNAL OF BACTERIOLOGY, vol. 124, no. 3, December 1975, pages 1067-1076, American Society for Microbiology, US; J.V. HÖLTJE et al.: "Novel type of murein transglycosylase in Escherichia coli" <br> * Page 1074, column 2; page 1075 * | 10 | **TECHNICAL FIELDS SEARCHED (Int. Cl.4)** <br><br> C 12 N <br> C 12 P |

The present search report has been drawn up for all claims

| Place of search | Date of completion of the search | Examiner |
|---|---|---|
| THE HAGUE | 13-01-1987 | CUPIDO M. |